(19) Europäisches Patentamt
European Patent Office
Office européen des brevets

(11) **EP 1 767 146 A1**

(12) **EUROPEAN PATENT APPLICATION**

(43) Date of publication:
**28.03.2007 Bulletin 2007/13**

(51) Int Cl.:
***A61B 5/0476*** (2006.01)    ***A61B 5/04*** (2006.01)

(21) Application number: **05020620.0**

(22) Date of filing: **21.09.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Max-Planck-Gesellschaft zur Förderung der Wissenschaften e.V.**
**80539 München (DE)**

(72) Inventors:
• **Becker, Klaus Dr.**
  **81369 München (DE)**

• **Eder, Matthias Dr.**
  **81669 München (DE)**
• **Ranft, Andreas Dr.**
  **82057 Icking (DE)**
• **Dodt, Hans-Ulrich Dr.**
  **80796 München (DE)**
• **Zieglgänsberger, Walter Prof. Dr.**
  **80939 München (DE)**

(74) Representative: **Hertz, Oliver**
  **v. Bezold & Partner**
  **Patentanwälte**
  **Akademiestrasse 7**
  **80799 München (DE)**

(54) **Monitoring neuronal signals**

(57)    A monitoring device (1) for monitoring an individual (2) under investigation comprises a recording device (10) for recording a neuronal signal from the individual (2), and a calculating device (20) for calculating a parameter representing a quantitative measure of an anaesthesia or coma condition of the individual (2) and being derived from the neuronal signal, wherein the calculating device (20) is adapted for calculating a complexity parameter representing the quantitative measure of the anaesthesia or coma condition, and the calculating device (20) includes a buffer circuit (21) for storing at least one time series of the neuronal signal and an analysis circuit (22) for subjecting the time series to a recurrence quantification analysis (RQA) providing the complexity parameter. Furthermore, a method of estimating a parameter representing a quantitative measure of an anaesthesia or coma condition of an individual is described.

FIG. 1

**Description**

Subject of the invention

The present invention relates to a monitoring device for monitoring neuronal signals, in particular for monitoring consciousness related brain conditions like anaesthesia or coma conditions. Furthermore, the present invention relates to a method of monitoring neuronal signals, in particular for calculating a complexity parameter characterizing the neuronal signals, like e. g. an anaesthetic parameter representing a quantitative measure of anaesthesia.

Prior art

Unconscious individuals do not respond to verbal command and other sensory stimulation, such as touch and pain. The clinical assessment of the cerebral (brain) function belongs to basic medical practice, and is applied e.g. for scoring trauma patients by means of the Glasgow Coma Scale. To enable major surgery, unconsciousness is induced pharmacologically by an anaesthesiologist (general anaesthesia). In case of pharmacologically induced unconsciousness, i. e., general anaesthesia for a surgical intervention, the patient's cerebral state is monitored by an anaesthesiologist.

Routine monitoring of general anaesthesia is based on clinical observation of the patient in combination with online measurement of cardiovascular parameters. More precisely, if surgical stimulation provokes neither movement nor increase in heart rate or blood pressure, it is likely that anaesthesia is sufficient. However, patients frequently receive peripherally acting muscle relaxants, preventing movement without affecting consciousness, so that the absence of movements is not significant anymore. In addition to that, in the many patients receiving betablockers, the cardiovascular response to painful stimuli is not a reliable predictor of insufficient anaesthesia either.

Moreover, in certain subgroups of patients, the dosage of anaesthetics may be restricted, like in obstetrics or cardiac surgery, resulting in a rather "light" anaesthesia. It is especially in such patients that intraoperative awareness can occur, leading to explicit memory of words spoken in the operating room, discomfort, or pain. Possible sequels of such explicit memory can be nightmares or even a posttraumatic stress disorder.

In addition to such memories accessible to conscious recall (explicit memory), implicit memories can be acquired under general anaesthesia. In this way, pain sensations, acoustic or other sensory input can influence the brain without being conscious to the individual. If the central processing of noxious stimuli is not sufficiently blocked, pain can be registered, leading to pain memory and possibly resulting in damage of nerve cells.

The right anaesthetic regimen would block such implicit memories without endangering or even harm the patient with an overdose. For this purpose, a device reliably measuring the cerebral function is required. The following techniques have been developed to derive a quantitative measure of anaesthesia from the electroencephalogram (EEG).

The raw EEG signal is processed by a Fast Fourier Transform and a power spectrum is calculated. The latter is then characterized by a number of parameters which are used to compose a single value between 0 (isoelectric EEG) and 100 (patient awake) .

One of these parameters is called bispectrum index (BIS) (Sebel et al. "A multicenter study of bispectral electroencephalogram analysis for monitoring anesthetic effect" in "Anesth. Analg." vol. 84, 1997, p. 891-899). The literature about an eventual benefit of BIS-monitoring is still equivocal. While some researchers have noted a reduction of anaesthetic dosing and shorter wake-up times, others could not confirm such advantages. Cases have been documented where the BIS monitor had indicated anaesthesia in an awake patient and, vice versa, deep anaesthesia has not been recognized as such. Besides, BIS values vary depending on the drugs used, and keta-mine-induced unconsciousness is not properly recognized at all.

As an alternative, an EEG signal evaluation with a combined analysis in the frequency and time domain has been developed (US 6 011 990). Parameters obtained from the EEG signal are subjected to a multivariate classification resulting in a dimensionless index. This method has a restricted reliability as well. Like BIS monitoring, the accuracy of anaesthesia quantification depended on the anaesthetic regimen.

Another approach evaluates a multi-channel EEG for obtaining a multivariate index (so-called patient state index, PSI) (Prichep et al. "Quantitative EEG assessment of changes in the level of the sedation/hypnosis during surgery under general anesthesia" IVth edition, Eds.: Jordan, Vaughan, Newton. London, Imperial College Press, 2000, pp 97-107). The drawback of this method is a low reliability and reproducibility, and a high complexity of calculation. And again, the choice of drugs influences the result.

A. C. Watt et al. ("Anaesthesiology" vol. 81, 1994, ASA Abstract A 478) have described another approach for characterizing anaesthesia, which is based on a correspondence of a so-called EEG dimensionality and an anaesthetic level. The dimensionality is a parameter of the EEG signals obtained from a strange attractor analysis. H. C. Watt et al. have found a non-linear relationship between the dose of anaesthetic and the EEG dimensionality. However, due to low reliability and the required processing power, this approach has not been applied in medical practice.

Another method for evaluating EEG signals is the recurrence quantification analysis. As an example, N. Thomasson et al. ("Physics Letters A", vol. 279, 2001, p. 94-101) have described the application of recurrence quantification analysis for evaluating epileptic EEG activity. With the RQA method, the detection of preictal EEG transients has been shown. As a further example, I.-H. Song et al. ("Neuroscience Letters" vol. 366, 2004, p. 148-153) have used the recurrence quantification analysis for analysing sleep EEG signals. These investigations have proved that sleep is a functional condition of the brain with high complexity being comparable with the complexity of the wake condition. Depending on the sleep phases, strong variations of EEG complexity with decreasing and increasing gradients have been found.

Generally, the conventional applications of recurrence quantification analysis of EEG signals have disadvantages in terms of a low significance of the results. Furthermore, the conventional techniques have allowed an off-line analysis of neuronal data only.

Object of the invention

The object of the invention is to provide an improved monitoring device for monitoring neuronal signals, in particular for monitoring anaesthesia or other consciousness related conditions of an individual, wherein the monitoring device is capable of avoiding the disadvantages of the conventional techniques. In particular, the monitoring device is to be improved with regard to the significance, reliability and reproducibility of monitoring neuronal signals. In particular, anaesthesia conditions are to be analysed independently on the type of anaesthesia or anaesthetic. Furthermore, the object of the invention is to provide an improved method of monitoring neuronal signals avoiding the disadvantages of the conventional techniques. In particular, a method of estimating a complexity parameter is to be provided.

Summary of the invention

These objects are solved by a monitoring device and a method with the features of claims 1 or 11, respectively. Advantageous embodiments and applications of the invention are defined in the dependent claims.

According to a first aspect, the present invention is based on the general technical teaching of providing a monitoring device for monitoring an anaesthesia or coma condition of an individual under investigation, preferably for anaesthesia monitoring, comprising a recording device for recording a neuronal signal and a calculating device for calculating a complexity parameter representing a quantitative measure of a consciousness condition on the individual, wherein the complexity parameter is calculated on the basis of at least one neuronal signal time series subjected to a recurrence quantification analysis (RQA). For providing the at least one neuronal signal time series, the monitoring device of the invention includes a buffer circuit for storing neuronal signal data.

Contrary to the conventionally used narcosis monitoring techniques based on a frequency analysis of EEG data, the monitoring device of the invention is adapted for an evaluation of the neuronal signals exclusively in the time domain. The inventors have found that depending on the type of anaesthetics used, frequency bands in the EEG signal can be increased or reduced. For this reason, the reliability of the conventional techniques has been found to be restricted. Contrary to the conventional concepts of characterizing a narcosis based on strange attractor theory, the present invention provides results with relative simple data processing technique (RQA), whereby an essentially increased reproducibility has been reached.

The present invention provides a signal processor being improved in terms of costs and simple structure. The application of RQA allows an essentially increased processing speed, which opens the application in practical medicine, like in controlling surgery. The monitoring device is adapted for a reliable on-line analysis of neuronal data. The complexity parameter is obtained in real time. Further advantages of applying RQA are derived from the fact that RQA is implemented on the basis of a set of RQA parameters, which can be directly adjusted at the monitoring device of the invention. The RQA applied according to the invention yields a complexity parameter which can be directly presented e.g. on a display and which can be used as a direct quantitative measure of the consciousness condition, e.g. anaesthesia condition of the individual under investigation.

Furthermore, contrary to the conventionally used techniques based on a spectral analysis of EEG signals, the complexity parameter obtained with the invention is independent on the anaesthetic used. The RQA can be applied in particular with high reproducibility with anaesthesias based on Ketamin, Propufol, Sevofluran. This anaesthetic type independency represents an essential advantage compared with the conventional techniques as the monitoring device of the invention allows a precise control of short-time narcosis treatments, in particular for ambulant surgery or with shallow narcosis treatments, e.g. with hearth surgery or births.

In the present specification, the term "anaesthesia" refers to a total or partial loss of sensation induced by disease or an anaesthetic, while the term "coma" refers to a deep, prolonged unconsciousness, e.g. induced by an injury, a disease or poison. Accordingly, the complexity parameter provided by the invention can be used for characterizing the depth of anaesthesia or coma conditions. The term "complexity parameter" refers to a quantity (preferably a single numeric value, e. g. in the range of 0 to 1 or 0% to 100 %) being a statistic measure of the randomness or probability of physiological (neuronal) processes determining an anaesthesia or coma condition.

According to a further important advantage, the monitoring device of the invention can be provided as compact equipment for routine operation in medical practice. The compact construction as a combination of standard components yields an essential cost reduction compared with conventional devices having costly spectral analysers. Furthermore, the monitoring device is adapted to requirements in medical applications in terms of easy use, cleaning and disinfection.

A further advantage of the invention is related to the fact that the monitoring device can be adapted for processing various types of neuronal signals, which comprise any type of electro-physiological data, which in particular can be recorded with conventional techniques. As examples, the neuronal signal may comprise EEG signals, EMG (electro myography) signals, or signals measured with the patch-clamp technique or derived from electro-physiological field potentials. According to a preferred embodiment of the invention, the monitoring device is adapted for processing EEG signals. This embodiment has particular advantages in terms of the available technology of EEG recording. EEG measurements represent a standard tool during surgery. Accordingly, the recording device used according to the invention may comprise simply an EEG recorder, which can be combined with a standard EEG sensor equipment.

According to a particularly preferred embodiment, the EEG recorder is adapted to be record one EEG signal only. Accordingly, the EEG recorder is connected with exactly two signal lines (measurement line/reference line) only. The inventors have found that one revulsion of electro-physiological data is enough for implementing the RQA according to the invention. The monitoring device does not necessarily require the recording of a complete EEG with e.g. 2 or more revulsions. Additionally, a ground line may be used for avoiding influences from the power supply system.

According to a further preferred embodiment of the invention, the monitoring device is adapted for evaluating the EEG signal in a predetermined frequency band only. For this purpose, the EEG recorder or the calculating device includes a filter circuit for recording or processing EEG data having frequencies in the selected frequency band. This embodiment of the invention has an advantage as data processing can be adapted to a frequency band with high contents of relevant information. Particularly preferred is the recording of EEG signals in the gamma-frequency band, which typically is not influenced by sleep effects. According to this preferred embodiment of the invention, RQA is implemented in another frequency band compared with the conventional investigations of sleep data. According to a further preferred embodiment of the invention, frequency components with frequencies above the gamma frequency band are recorded. The inventors have found that in the high-frequency range (above 20 Hz), complexity parameters can be obtained with high reproducibility and low signal-to-noise-ration (SNR).

The monitoring device according to the invention includes an analysis circuit being adapted for implementing the recurrence quantification analysis. Depending on the application of the monitoring device, the analysis circuit can be provided with a fixed set of RQA parameters yielding an advantage in terms of simple structure of the monitoring device and increased processing speed. However, according to a preferred embodiment of the invention, the analysis circuit is adapted for setting RQA parameters. This embodiment allows an improved adaptation of the monitoring device to the practical application. Preferably, the analysis circuit includes an adjustment device for setting the RQA parameters. The adjustment device can be adapted for manually setting the RQA parameters by an operator or for automatically setting the RQA parameters in dependence on the operation conditions of the monitoring device. In the latter case, preferably a control loop is implemented for controlling of the adjustment device in dependence on the output of the calculating device.

According to a further advantageous embodiment of the invention, the calculating device may include at least one of an evaluating device for further processing the complexity parameter and an alarm device for indicating predetermined alarm conditions of the complexity parameter or any other value obtained from the complexity parameter by further processing.

According to a second general aspect, the present invention provides an anaesthesia device for surgery anaesthesia, which includes the above monitoring device according to the invention. Furthermore, the anaesthesia device includes an anaesthesia control device for controlling anaesthesia parameters, like the type of anaesthetic or the time of supplying a certain anaesthetic. The anaesthesia device of the invention has an essential advantage in terms of being suitable for an automatic or semi-automatic anaesthesia. In this case, the anaesthetist is required for monitoring the anaesthesia device only, while essential functions, like e.g. dosing the anaesthetic is provided automatically.

According to a further general aspect, the present invention is based on the general technical teaching of providing a method of estimating a parameter representing a quantitative measure of anaesthesia or coma, wherein time series of a neuronal signal from an individual under investigation are subjected to a recurrence quantification analysis yielding a complexity parameter. RQA has been found as being a significant tool for determining the regularity or complexity of time series of neuronal signals.

Basic principles and theory of RQA have been developed and published by C. L. Webber et al. in "J. Appl. Physiol." vol.76, 1994, p. 965-973. The time series with a predetermined number of values are represented in a phase space with a predetermined embedding dimension m. The variation of the neuronal signal, in particular of a recorded EEG signal yields a trajectory of the current state in the phase space. The complexity parameter measured with RQA is a quantitative characterization of the trajectory movement in the phase space. Strong variations of the trajectory represent a high complexity, while low variations represent a low complexity. The variation of the trajectory or a value derived from this variation can be used for a direct classification of the complexity parameter.

The present inventors have found preferred ranges of the RQA parameters embedding dimension (m), time delay (d), matrix threshold (r) and minimal line length (L). According to a preferred embodiment of the invention, the embedding dimension m (number of values of each time series) is selected to be in the range of 10 to 30. This embedding dimension range represents advantages in terms of processing speed and reproducibility. Furthermore, the parameter time delay used for the subsequent construction of time series is preferably selected in the range of 1 to 20. With matrix threshold (r) in the range of 0,1 to 0,6, a suitable sensitivity in particular for anaesthesia monitoring has been obtained. Minimal line length (L) in the range of 50 to 500 has been proved to result in high stability and reproducibility.

Another independent aspect of the invention is represented by the application of the monitoring device or the method according to the invention for long-term monitoring brain functions of a patient or for monitoring and/or controlling anaesthesia induced by an anaesthetic during a surgical operation.

Brief description of the drawings

Further details and advantages of the invention will be described in the following with reference to the attached drawings, which show:

Figure 1    a schematic illustration of a monitoring device according to an embodiment of the invention,

Figure 2    a schematic illustration of further details of the monitoring device according to Figure 1,

Figure 3    further details of a practical embodiment of a monitoring device,

Figure 4    a flow diagram illustrating the steps of a method according to the invention,

Figure 5    a flow diagram illustrating further details of the method according to the invention,

Figure 6    a curve chart illustrating experimental results obtained with the invention, and

Figure 7    further curve charts illustrating experimental results obtained with the invention.

Embodiments of the invention

A preferred embodiment of the invention is described in the following with reference to anaesthesia monitoring during surgery. Details of medical equipment used for EEG recording and anaesthesia are not described as far as they are known from conventional medicine techniques. Furthermore, it is emphasized that the invention can be implemented in an analogue way with alternative applications for monitoring anaesthesia induced by a disease or monitoring coma patients.

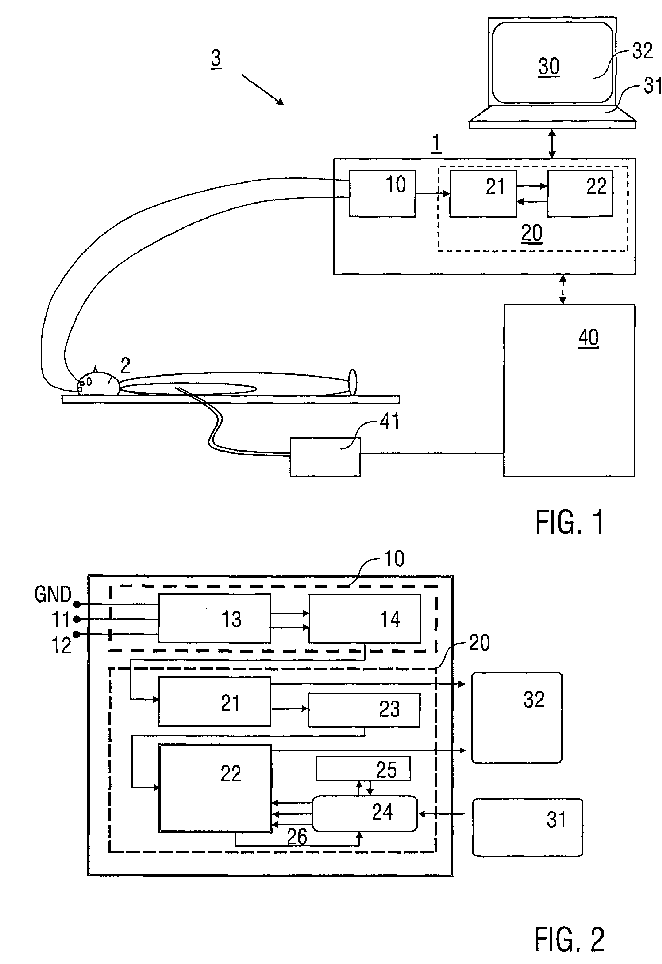

Figure 1 schematically illustrates an embodiment of the monitoring device 1 with an EEG recorder 10 and a calculating device 20 including a buffer circuit 21 for storing at least one time series of EEG signals and an analysis circuit 22 for analysing the EEG signal with recurrence quantification analysis. The EEG recorder has a structure like a conventional recorder device. The calculating device 20 comprises a standard board with processors implementing the calculation and adjustment functions outlined below.

The EEG recorder 10 is connected via two signal lines 11, 12 with EEG sensors to be applied on the head of an individual 2 under investigation. The lines comprise a measurement line and a reference line. Additionally, a ground line is provided. The monitoring device 1 is equipped with a control unit 30 including an input interface 31 and a display 32. Components 10, 20 and 30 are arranged within a common casing. The complete anaesthesia device 3 according to the invention comprises the monitoring device land an anaesthesia control device 40 controlling an anaesthetic supply unit 41.

Further details of the monitoring device 1 are illustrated in Figure 2. The EEG recorder 10 comprises an EEG amplifier 13 and an A/D-converter 14. The input of the EEG amplifier is connected with the signal lines 11, 12 connected with a signal electrode and a reference electrode on the patient as well as with a ground electrode. EEG signals amplified with the EEG amplifier 13 are digitised with the A/D-converter 14. The digital EEG signals are output to the calculating circuit 20.

The calculating circuit 20 comprises the buffer circuit 21, the analysis circuit 22, the filter circuit 23 and an adjustment circuit 24. These components are arranged on a dual processor computer main board. The digital EEG data are stored in the buffer circuit 21. The filter circuit 23 comprises a digital FIR-filter. EEG data having frequency components in a predetermined frequency range of e.g. 20 Hz to 150 Hz are delivered via the filter circuit 23 to the analysis circuit 22, which is adapted for the calculation of the complexity values as outlined below. This calculation comprises the recurrence quantification analysis implemented with certain RQA parameters, which are supplied from the adjustment circuit 24 to the analysis circuit 22. The RQA parameters can be stored in a further parameter memory 25 or set by an operator via the input interface 31, e.g. by using optical encoders. Furthermore, a control loop 26 can be implemented for varying RQA parameters in dependence on the result of the complexity value calculation. The raw data stored in the memory circuit 21 and the complexity parameter calculated in the analysis circuit 22 are output to the control unit and displayed on the display device 32, which comprises e.g. a LCD display.

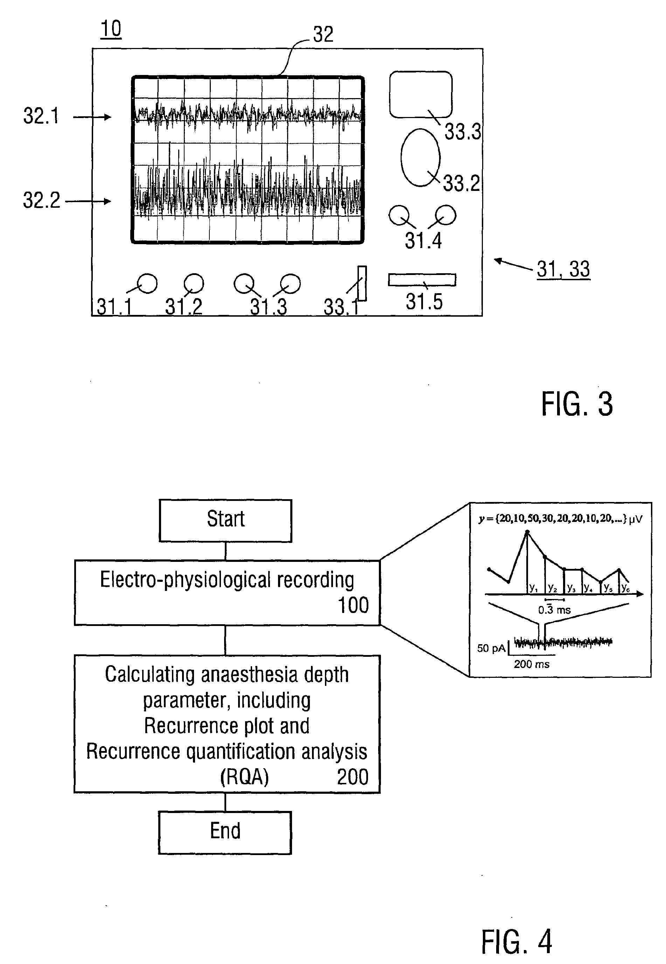

The components 10, 20 and 30 shown in Figure 1 are preferably provided in a single compact device. An example of a front panel of a compact monitoring device 10 according to the invention is illustrated in Figure 3. The front panel includes the input interface 31 and the display 32 as shown in Figure 1. The input interface 31 comprises a power switch 31.1, a radius adjustment element 31.2, scaling elements 31.3 for scaling the traces 32.1 and 32.2 of the display 32, and velocity trace elements 31.4. In the upper trace 32.1 represented in display 32, the raw data of the EEG signal are shown, while the lower trace 32.2 represents the calculated complexity parameters.

Additionally, the front panel serves as an output interface 33 with a digital data output 33.1, loud speaker 33.2 connected with an alarm device and a numerical representation 33.3 e.g. of the actual complexity parameter and a further reference parameter, like e.g. an average complexity parameter. The reference numeral 31.5 refers to the connector for accommodating the signal and ground lines 11, 12 and GND illustrated in Figure 2.

The operation principles of the monitoring device according to the invention are described in the following with exemplary reference to the calculation of an anaesthesia depth parameter (complexity parameter) as shown in Figures 4 to 6. According to Figure 4, the method of the invention comprises the steps of electro-physiological recording of a neuronal signal, in particular an EEG signal (step 100) and the step of calculating an anaesthesia depth parameter on the basis of RQA (step 200).

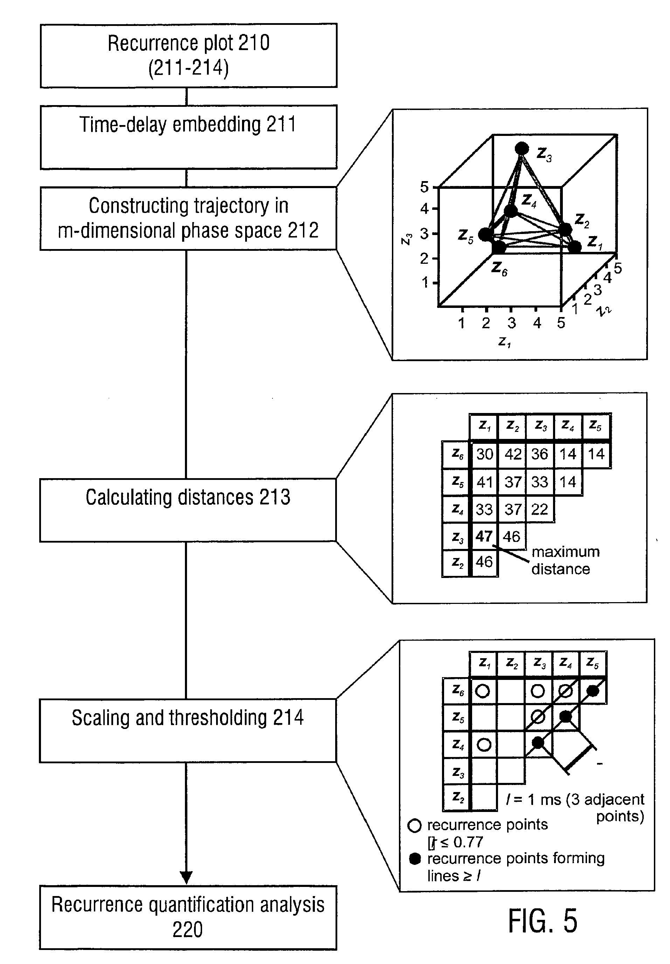

With step 100, time series of discrete EEG voltage or current values are recorded. Each time series represents a segment of measured values with a length of e.g. 1000 samples. The time series (segments) are stored in the memory circuit 21 (see Figure 2) without overlap in time. The sample frequency is selected in the range of 500 Hz to 2 kHz, e.g. 1 kHz. The insert of Figure 4 illustrates the measurement of current values. The measured values being sampled with a time interval of 1 ms yield the time series y indicated in the upper part of the insert. The following calculating step 200 comprises the recurrence plot 210 with sub-steps 211 to 214 and the recurrence quantification analysis 220 as illustrated in Figure 5.

As the first sub-step 211, the recorded time series are subjected to time-delay embedding. Time-delay embedding comprises a substitution of each measured value by a vector with the embedding dimension m. According to the theorem of Takens, the embedding vector **Z** comprises the components $z_i$ according to:

$$z_i = y_i, \; y_i + \tau, \; \ldots, \; y_i + (m-1)\tau$$

with m being the embedding dimension and $\tau$ being the time delay parameter.

While embedding dimension m used in practice is preferably selected with e. g. m = 10 or m = 20, the present illustration refers to the parameters m = 3 and $\tau$ = 0.3 ms for clarity reasons. With these parameters, the measured values are replaced by a series of time delay vectors:

$$z = \left\{ \begin{pmatrix} 50 \\ 10 \\ 20 \end{pmatrix} \bullet \begin{pmatrix} 30 \\ 50 \\ 10 \end{pmatrix} \bullet \begin{pmatrix} 20 \\ 30 \\ 50 \end{pmatrix} \bullet \begin{pmatrix} 20 \\ 20 \\ 30 \end{pmatrix} \bullet \begin{pmatrix} 10 \\ 20 \\ 20 \end{pmatrix} \bullet \begin{pmatrix} 20 \\ 10 \\ 20 \end{pmatrix} \bullet \ldots \right\} \mu V$$

Each vector can be considered as representing a specific point in the phase space. As the next sub-step, the time-delay vectors are used for constructing a trajectory in the m-dimensional phase space (step 212 in Figure 5). The sequence of vectors forms a trajectory as exemplary illustrated in the upper insert of Figure 5. The trajectory is characterized by a random variation ("jumping") in time. The RQA applied according to the invention is used for quantitatively characterizing the degree of variation of the trajectory in phase space, which is considered as the complexity parameter for describing the consciousness condition of the brain during anaesthesia or coma.

Subsequently, the distances of the points in phase space are calculated with the next sub-step 213. The distances of all points $Z_i$ relative to each other are estimated and represented in a triangular matrix (table) as shown in the insert of Figure 5. According to a first alternative, the distances are calculated with the euclidic metric:

$$dist(z_i, z_j) = \sqrt{\sum_{k=1}^{m} (z_{i,k} - z_{j,k})^2}$$

According to a second alternative, the "city-block"- or Manhattan-metric is used for calculating the distances according to:

$$dist(z_i, z_j) = \sum_{k=1}^{m} (z_{i,k} - z_{j,k})$$

The euclidic metric has an advantage in terms of precise calculation of the distances. However, the "city-block"-metric is preferred as the inventors have obtained results with high precision (comparable with euclidic metric) while the processing speed is increased and the processing costs is essentially reduced.

With the next sub-step 314, a scaling and thresholding is implemented. Scaling comprises a standardisation of the calculated distances. According to a first alternative, the standardisation is implemented relative to an average distance (so-called average scaling). According to a second alternative, the standardisation is implemented relative to a maximum distance obtained in phase space (so-called maximum-scaling). For calculating complexity parameters according to the invention, the application of maximum-scaling is preferred. As a result, scaled distances in the range of 0 to 1 are obtained. With the illustrated example, all elements of the triangular matrix are divided by 47.

According to a further alternative, standardisation can be omitted in step 314. In this case, the further calcultation is implemented directly with the distance measure (ms).

Subsequently, all scaled distance values are subjected to a matrix thresholding which comprises a digitalisation wherein all scaled distance values equal or above a predetermined threshold value r are set to 1, while all remaining distance values below r are set to 0.

The matrix threshold value r is selected depending on the particular application of the invention. For evaluating EEG signals, r = 0.4 has been found to be suitable. Preferably, the threshold value is adjustable for optimising the sensitivity of the method to the requirements of a particular application of the invention.

The result of the matrix thresholding of scaled distance values is illustrated in the insert (bottom) of Figure 5. All points below the matrix threshold value r (set to 0) are illustrated with circles while the other values (set to 1) are omitted. The graphical representation illustrated in the insert (bottom) of Figure 5 represents a recurrence plot, which in practice has an essentially more complex appearance.

On the basis of the recurrence plot, the recurrence quantification analysis (step 220) is implemented as follows. The recurrence plot includes points forming diagonal lines (full circles). The length of these lines is characteristic for time ranges in which the trajectory of the system has low variations only. In these time ranges, the system has a low complexity or dynamic (relative stable condition). The RQA comprises a further thresholding with a so-called minimal line length parameter L (minimal line length thresholding). All points in the recurrence plot forming a diagonal line with a length greater than L are estimated. In practice a minimal line length parameter L = 100 is preferred.

Finally, the ratio of points fulfilling the minimal line length thresholding condition and the complete number of points in the recurrence plot (fulfilling the above scaled value thresholding condition) is calculated. The complexity parameter to be obtained with the method of the invention is represented by the determinism parameter D [%] according to:

$$D = \frac{\text{number of recurrence points forming lines} \geq l}{\text{total number of recurrence points}} \times 100$$

Determinism D is a quantitative measure for the predictability or regularity of the time series measured. For the application in anaesthesia monitoring, the calculation of the complexity K according to

$$K = -\log(D)$$

is preferred. The value K = 0 would represent a perfect regularity, while higher values indicate a more complex behaviour. For providing a complexity parameter K' with dB scale, K' is calculated as -20 log(D).

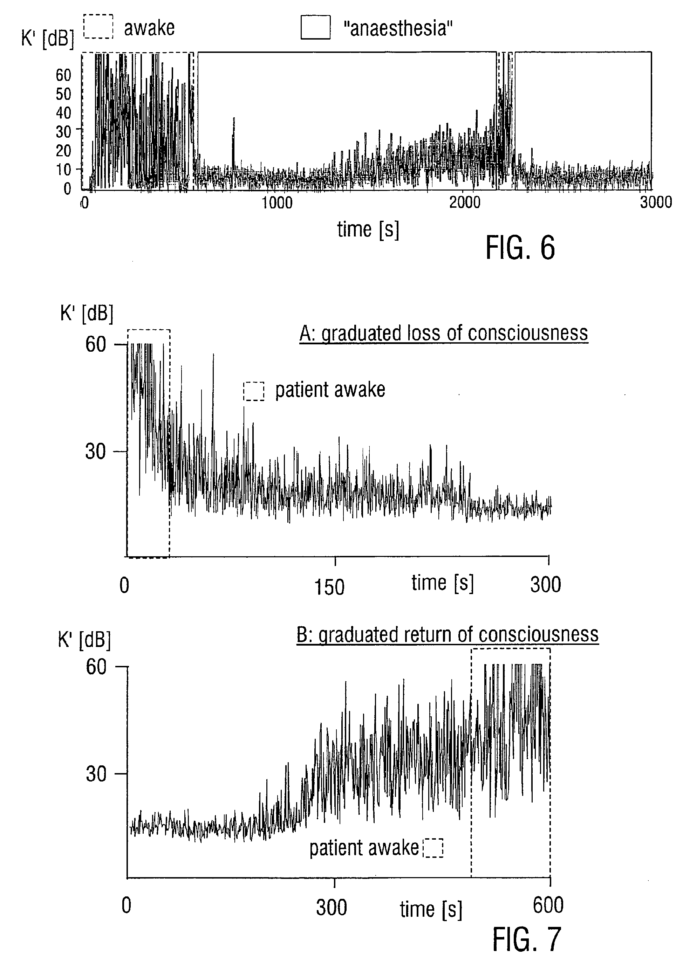

Figure 6 illustrates experimental results of RQA with EEG signals. The inventors have found various phases with high D values and essentially reduced D values, respectively. These different phases ("awake" and "anaesthesia") have been correlated with different conditions of a patient obtained with monitoring vegetative parameters of the individual indicating awake and anaesthesia conditions corresponding to high and low D values, respectively.

The results of the inventors have shown that the parameter K has a pronounced dependency on time. After injection of an anaesthetic, the K value is decreased within a range of 1 to 2 min down to a constant value (see Figure 7A). This K gradient in time has been found with high reproducibility. On the other hand, the K value is increased after completing the anaesthetic supply within a range of about 10 to 20 min (see Figure 7B). After this phase of increasing the K value, a K value jump has been found being correlated with the wake-up of the individual.

The reproducible time gradients with decreasing and increasing K values and the reproducible K value jump allow a calibration of the K value profiles obtained with an individual. On the basis of this calibration, a quantitative measure for narcosis depth has been found, which is reproducible and suitable for describing various phases of anaesthesia.

On the basis of the anaesthesia parameter obtained with the invention, the anaesthesia control device 40 (Figure 1) can be set for supplying the anaesthetic supply 41 for obtaining a predetermined degree of narcosis.

## Claims

1. Monitoring device (1) for monitoring an individual (2) under investigation, comprising:

   - a recording device (10) for recording a neuronal signal from the individual (2), and
   - a calculating device (20) for calculating a parameter representing a quantitative measure of an anaesthesia or coma condition of the individual (2) and being derived from the neuronal signal,

   **characterized in that**

   - the calculating device (20) is adapted for calculating a complexity parameter representing the quantitative measure of the anaesthesia or coma condition, and
   - the calculating device (20) includes a buffer circuit (21) for storing at least one time series of the neuronal signal and an analysis circuit (22) for subjecting the time series to a recurrence quantification analysis (RQA) providing the complexity parameter.

2. Monitoring device according to claim 1, wherein the recording device (10) includes an EEG recorder (10) for recording an EEG signal being the neuronal signal from the individual (2).

3. Monitoring device according to claim 2, wherein the EEG recorder (10) includes two signal lines (11, 12) to be connected with the individual (2).

4. Monitoring device according to claim 2, wherein the EEG recorder (10) or the calculating device (20) includes a filter circuit (23) for recording frequency components in a predetermined frequency band of the EEG signal.

5. Monitoring device according to claim 4, wherein the filter circuit (23) is adapted for recording frequency components in the gamma-frequency band of the EEG signal.

6. Monitoring device according to at least one of the foregoing claims, wherein the analysis circuit (22) includes an adjustment device (24) for setting RQA parameters of the RQ analysis.

7. Monitoring device according to claim 6, wherein the calculating device (20) includes a control loop (25) for controlling the adjustment device (24).

8. Monitoring device according to at least one of the foregoing claims, wherein the calculating device (20) includes an evaluation device (26) for further processing the complexity parameter.

9. Monitoring device according to claim 8, wherein the evaluation device (26) includes an alarm device (27).

10. Anaesthesia device (3) comprising a monitoring device (1) according to at least one of the foregoing claims.

11. Method of estimating a parameter representing a quantitative measure of an anaesthesia or coma condition of an individual (2), comprising the steps of:

>    - recording (100) of a neuronal signal from an individual (2) under investigation, and
>    - calculating (200) the quantitative measure of the anaesthesia or coma condition

**characterized in that**

>    - the recording step includes the step of storing at least one time series of the neuronal signal, and
>    - the calculating step includes the step of subjecting the time series to a recurrence quantification analysis (RQA) for providing a complexity parameter being derived from the neuronal signal.

12. Method according to claim 11, wherein the neuronal signal comprises an EEG signal and the recording step includes the step of recording the EEG signal from the individual (1).

13. Method according to claim 12, wherein frequency components in a predetermined frequency band of the EEG signal are recorded.

14. Method according to claim 13, wherein frequency components in the gamma-frequency band of the EEG signal are recorded.

15. Method according to at least one of the claims 11 to 14, wherein the stored time series of the neuronal signal comprises at least 500 signal values.

16. Method according to at least one of the claims 11 to 15, further comprising the step of setting at least one RQA parameter, wherein the RQA parameter comprises at least one embedding dimension (m), time delay (d), matrix threshold (r) and minimal line length (L).

17. Method according to claim 16, wherein at least one of the RQA parameters is set in the following ranges:

>    - embedding dimension (m) in the range of 10 to 30,
>    - time delay (d) in the range of 1 to 20 s,
>    - matrix threshold (r) in the range of 0,1 to 0,6, and
>    - minimal line length (L) in the range of 50 to 500.

18. Method according to at least one of the claims 11 to 17, wherein the RQA includes a step of calculating distances of phase space vectors on the basis of a city-block-metric.

19. Method according to at least one of the claims 11 to 18, wherein the RQA includes a step of normalizing the calculated distances relative to a maximum distance value.

20. Method according to at least one of the claims 11 to 19, wherein the complexity parameter comprises a complexity value K obtained from an RQ determinism value D according to $K = - \log D$.

21. Method according to at least one of the claims 11 to 20, further comprising the step of evaluating the complexity parameter.

22. Method of using monitoring device (100) or a method according to at least one of the foregoing claims, for:

>    - long term monitoring brain functions of a patient,
>    - monitoring and/or controlling anaesthesia during a surgical operation.

FIG. 1

FIG. 2

10

32

32.1 →

32.2 →

33.3

33.2

31.4

31, 33

31.1    31.2    31.3    33.1    31.5

## FIG. 3

Start

Electro-physiological recording
100

$y = \{20,10,50,30,20,20,10,20,...\}\,\mu V$

$y_1$  $y_2$  $y_3$  $y_4$  $y_5$  $y_6$

0.3 ms

50 pA

200 ms

Calculating anaesthesia depth
parameter, including
Recurrence plot and
Recurrence quantification analysis
(RQA)          200

End

## FIG. 4

FIG. 5

FIG. 6

A: graduated loss of consciousness

B: graduated return of consciousness

FIG. 7

**European Patent Office** | **PARTIAL EUROPEAN SEARCH REPORT** | **Application Number**

which under Rule 45 of the European Patent Convention shall be considered, for the purposes of subsequent proceedings, as the European search report

EP 05 02 0620

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 442 421 B1 (LE VAN QUYEN MICHEL ET AL) 27 August 2002 (2002-08-27)<br>* abstract; figures 1-3 *<br>* column 2, line 29 - column 4, line 40 *<br>* column 4, line 53 - column 8, line 10 *<br>----- | 1-10 | A61B5/0476<br>A61B5/04 |
| Y | US 2002/173729 A1 (VIERTIO-OJA HANNA ET AL) 21 November 2002 (2002-11-21)<br>* abstract; figures 1-4 *<br>* paragraph [0020] - paragraph [0028] *<br>* paragraph [0035] - paragraph [0069] *<br>----- | 1-10 | |
| D,Y | SONG IN-HO ET AL: "Recurrence quantification analysis of sleep electoencephalogram in sleep apnea syndrome in humans."<br>NEUROSCIENCE LETTERS. 12 AUG 2004, vol. 366, no. 2,<br>12 August 2004 (2004-08-12), pages 148-153, XP002371520<br>ISSN: 0304-3940<br>* the whole document *<br>-----<br>-/-- | 1-10 | |

| | TECHNICAL FIELDS SEARCHED (IPC) |
|---|---|
| | A61B |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 10 March 2006 | Wetzig, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent Office**

**PARTIAL EUROPEAN SEARCH REPORT**

Application Number

EP 05 02 0620

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| A | US 6 547 746 B1 (MARINO ANDREW A) 15 April 2003 (2003-04-15) * the whole document * ----- | 1-10 | |
| D,A | US 6 011 990 A (SCHULTZ ET AL) 4 January 2000 (2000-01-04) * the whole document * ----- | 1-10 | |

TECHNICAL FIELDS SEARCHED (IPC)

EPO FORM 1503 03.82 (P04C10)

**European Patent Office**

**INCOMPLETE SEARCH SHEET C**

Application Number

EP 05 02 0620

Claim(s) not searched:
11-22

Reason for the limitation of the search (non-patentable invention(s)):

Article 52 (4) EPC - Method for treatment of the human or animal body by therapy

## ANNEX TO THE EUROPEAN SEARCH REPORT
## ON EUROPEAN PATENT APPLICATION NO.

EP 05 02 0620

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2006

| Patent document cited in search report | | Publication date | Patent family member(s) | | Publication date |
|---|---|---|---|---|---|
| US 6442421 | B1 | 27-08-2002 | US | 2002095099 A1 | 18-07-2002 |
| US 2002173729 | A1 | 21-11-2002 | EP<br>WO<br>JP | 1389953 A1<br>02094099 A1<br>2004527328 T | 25-02-2004<br>28-11-2002<br>09-09-2004 |
| US 6547746 | B1 | 15-04-2003 | NONE | | |
| US 6011990 | A | 04-01-2000 | AT<br>WO<br>DE<br>EP<br>ES<br>JP | 177548 T<br>9715013 A2<br>19538925 A1<br>0856181 A2<br>2131974 T3<br>11514901 T | 15-03-1999<br>24-04-1997<br>24-04-1997<br>05-08-1998<br>01-08-1999<br>21-12-1999 |

EPO FORM P0459

For more details about this annex : see Official Journal of the European Patent Office, No. 12/82

## REFERENCES CITED IN THE DESCRIPTION

### Patent documents cited in the description

- US 6011990 A

### Non-patent literature cited in the description

- **SEBEL et al.** A multicenter study of bispectral electroencephalogram analysis for monitoring anesthetic effect. *Anesth. Analg.,* 1997, vol. 84, 891-899
- **PRICHEP et al.** Quantitative EEG assessment of changes in the level of the sedation/hypnosis during surgery under general anesthesia. Imperial College Press, 2000, 97-107
- **A. C. WATT et al.** *Anaesthesiology,* 1994, vol. 81, 478
- **N. THOMASSON et al.** *Physics Letters A,* 2001, vol. 279, 94-101
- **I.-H. SONG et al.** *Neuroscience Letters,* 2004, vol. 366, 148-153
- **C. L. WEBBER et al.** *J. Appl. Physiol.,* 1994, vol. 76, 965-973